# EUROPEAN PATENT SPECIFICATION

(11) **EP 2 108 313 B1**
(45) Date of publication and mention of the grant of the patent: **17.08.2011**
(21) Application number: 08425247.7
(22) Date of filing: 11.04.2008
(51) Int. Cl.: A61B 7/00

(54) **Improved apparatus for determining dysphagia**
Verbesserte Vorrichtung zur Diagnose von Dysphagie
Appareil amélioré de diagnostic de dysphagie

(43) Date of publication of application: 14.10.2009
(73) Proprietor: Talluri, Antonio, 50136 Bagno a Ripoli, Firenze (IT); Weir, Robert James, London W60PF (GB)
(72) Inventor: Talluri, Antonio, 50136 Bagno a Ripoli, Firenze (IT); Weir, Robert James, London W60PF (GB)
(74) Representative: Mannucci, Michele

(56) References cited:
- JP-A- 2003 111 748
- JP-A- 2005 304 890
- JP-A- 2006 263 299
- JP-A- 2007 202 939
- KLEY C ET AL: "Diagnostic use of swallowing sounds in dysphagia; Sind Schluckgeräusche diagnostisch nutzbar?" DER NERVENARZT ; ORGAN DER DEUTSCHEN GESELLSCHAFT FÜR PSYCHIATRIE, PSYCHOTHERAPIE UND NERVENHEILKUNDE ORGAN DER DEUTSCHEN GESELLSCHAFT FÜR NEUROLOGIE, SPRINGER, BERLIN, DE, vol. 76, no. 12, 1 December 2005 (2005-12-01), pages 1495-1505, XP019321118 ISSN: 1433-0407

## Description

The present invention relates to an apparatus for determining dysphagia

People affected by dysphagia have difficulty in swallowing and may also experience pain while swallowing. Some people may be completely unable to swallow or may have trouble in swallowing liquids, foods or saliva. Often, dysphagia makes it difficult to take in enough calories and fluids to nourish the body. During the first stage of swallowing the tongue collects the prepared food and/or liquid, making it ready for swallowing. The second stage begins when the tongue pushes the food or liquid to the back of the mouth, which triggers a swallowing reflex that passes the food through the pharynx (the canal that connects the mouth with the esophagus). During this stage, the larynx (the canal connecting the mouth with the trachea and bronchi) closes hermetically and breathing stops to prevent food or liquid from entering the lungs. The third stage begins when the food or liquid enters the esophagus, the canal that carries food and liquid to the stomach. This passage through then esophagus usually occurs in three seconds, depending on the texture or consistency of the food.

Dysphagia is intended as the presence of a problem with any one of the stages of the swallowing process. A weak tongue, or weak cheek muscles, may make it difficult to move food around inside the mouth for chewing. Food pieces that are too large to be swallowed may enter the throat and block the passage of air. Other problems include the inability to start the swallowing reflex (a stimulus that allows a person to make food and liquid pass safely through the pharynx) because of a stroke or other nervous system disorder. People with these kinds of problems are unable to begin the muscle movements that allow food to move from the mouth to the stomach. Another difficulty can occur when weak throat muscles cannot move all of the food toward the stomach. Pieces of food can fall or be pulled into the trachea causing coughing and possible lung infection.

Dysphagia can be a serious disorder. Those who cannot swallow well may not be able to eat enough food to stay healthy or maintain an ideal weight. Sometimes, when foods or liquids enter the larynx of a person who has dysphagia, coughing or throat clearing cannot remove it. Food or liquid that remains in the larynx may enter the lungs and create a chance for harmful bacteria to grow. A serious infection (aspiration pneumonia) can result.

Swallowing disorders may also include the development of a pocket outside the esophagus caused by weakness in the esophageal wall. This abnormal pocket traps some of the food swallowed. While lying down or sleeping, a person with this problem may draw undigested food into the pharynx. The esophagus may be too narrow, causing food to stick. This food may prevent other food or even liquids from entering the stomach.

Current system for the diagnosis of dysphagia comprise: barium swallow X-ray, cervical auscultation, double-contrast barium enema, double-contrast upper GI, electrogastrography, EMG spectral analysis, Endoscopy, gastrointestinal endoscopic image atlas, fiber optic endoscopic evaluation of swallowing FEESST, Gastroscopy, measurement of muscle strength, Manometry, pH probe, Ultrasound, Upper GI for GER and others. In general these are invasive techniques which require complex devices.

The European Patent Publication N° 2005304890 discloses a method of detecting dysphagia by positioning electrodes on the patient's neck surface, recording an electromyogram and comparing it with data of normal persons. This procedure and the related test interpretation have to be conducted by trained peoples.

The document "Diagnostic use of swallowing sounds in dysphagia; Sind Schluckgeräusche diagnostisch nutzbar?" DER NERVENARTZ; ORGAN DER DEUTSCHEN GESELLSCHAFT FÜR PSYCHIATRIE, PSYCOTHERAPIE UND NERVENHEILKUNDE ORGAN DER DEUTSCHEN GESELLSCHAFT FÜR NEUROLOGIE, by KLEY C et AL., SPRINGER, BERLIN, DE, vol. 76, n°12, 1 December 2005 (2005-12-01), pages 1495-1505, XP01931118 ISSN: 1433-0407, discloses a simple and non-invasive procedure suitable to detect the presence of abnormal swallowing in a patient, in order to perform an initial selection of patients and therefore to subject only those patients for whom the procedure indicates the presence of abnormal conditions to specific tests of the type listed above.

According to the above document, the transit of liquid or solid boluses produces a sound that can be detected and analyzed to provide typical recordings in relation to swallowing disorders and a test procedure is suggested comprising the following steps:
- fitting, in proximity of the patient's larynx, a microphone acoustically isolated from ambient noise and connected to a recording/viewing system suitable to display, for example on a monitor, a recording of the sound intensity detected by the microphone;
- making the patient swallow boluses of various kinds, for example liquid boluses, and simultaneously recording and viewing said sound recording;
- comparing the recording obtained with standard recordings to identify the presence of abnormalities.

The invention is defined in claim 1.

The present invention as the purpose of providing an improved fastenable collar to be used in the suggested procedure and comprising a body made of sound insulating material to be positioned over the larynx which, on the inner surface thereof, has a cavity in which a microphone is inserted. The collar is intended to be fitted around the neck with the microphone positioned in contact with the skin of the neck at the height of the larynx, in a median position, and then connected to a recorder, such as a digital recorder.

According to the invention, the collar has a body made of sound insulating material with a symmetrical shape with respect to the vertical plane of symmetry of the user and with a part projecting downward which must be located in a central position on the patient's chest. This facilitates correct positioning of the collar.

The finding will be better understood by following the description and accompanying drawing, which shows a practical non-limiting example of said finding. In the drawing:
Fig.1 shows a collar provided with microphone according to the invention;
Fig.2 shows a section of the collar according to II-II of Figure 1;
Fig.3 schematically shows a patient with the collar worn correctly; and
Fig.4 shows an example of sound intensity recording, detected during the swallowing of a liquid bolus.

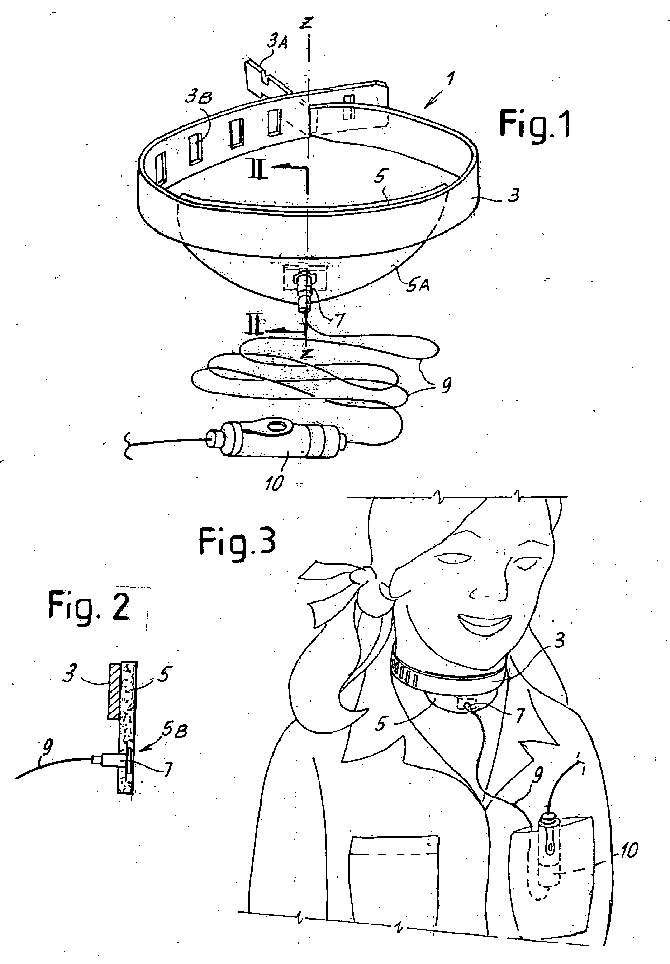

With reference to the drawing, a collar 1 (Fig.1) according to the invention comprises a strap 3, which is flexible and adjustable by inserting an end 3A into one of several holes 3B produced in the other end, and a strip 5 of expanded and flexible material secured to the strap 3 by gluing. The strip 5 projects downwardly from the strap 3 in the form of a rounded extension 5A symmetrical with respect to a vertical plane with the line Z-Z of symmetry of the user.

A recess 5B (Fig.2) with through hole, is produced in the extension 5A, suitable to receive a microphone 7 connectable to a digital recorder (not shown in the drawing) by means of a wire 9 provided with a push button switch 10 to activate and deactivate the microphone. The expanded material forming the strip 5 is suitable to acoustically isolate the microphone 7 from the external environment.

The digital recorder is in turn connected to a computer provided with specific software suitable to analyze the sound recordings detecting during a patient's test.

The downward pointed shape of the collar allows easy centering and positioning of the microphone in proximity of the glottis. Fig.3 shows the collar worn correctly, with the microphone 7 directly facing the patient's glottis.

For a dysphagia test, the patient is asked to swallow some boluses, for example and in particular liquids, continuously recording the sound. The recording is transferred to a computer which, by means of suitable programs, analyzes the acoustic spectrum and displays it on a monitor.

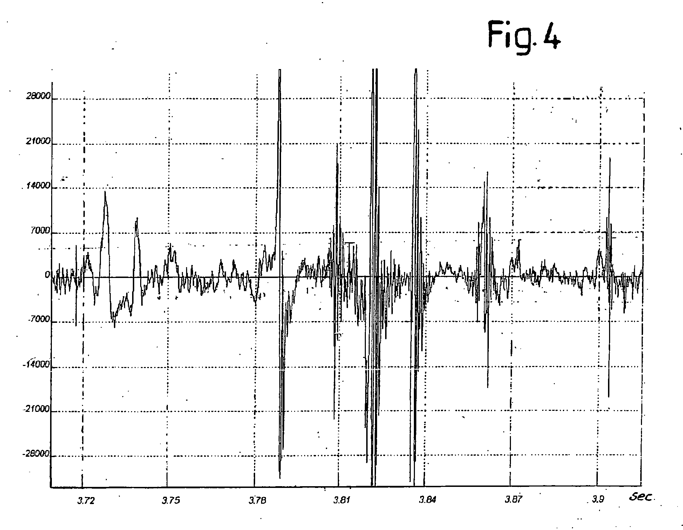

Fig.4 shows a typical recording obtained during the swallowing of a liquid bolus by a normal subject, the recording showing sound intensity on the ordinate and time on the abscissa: a series of sound pulses of short duration (10-20 ms) comparable to rapid hermetic closing of a valve is evident.

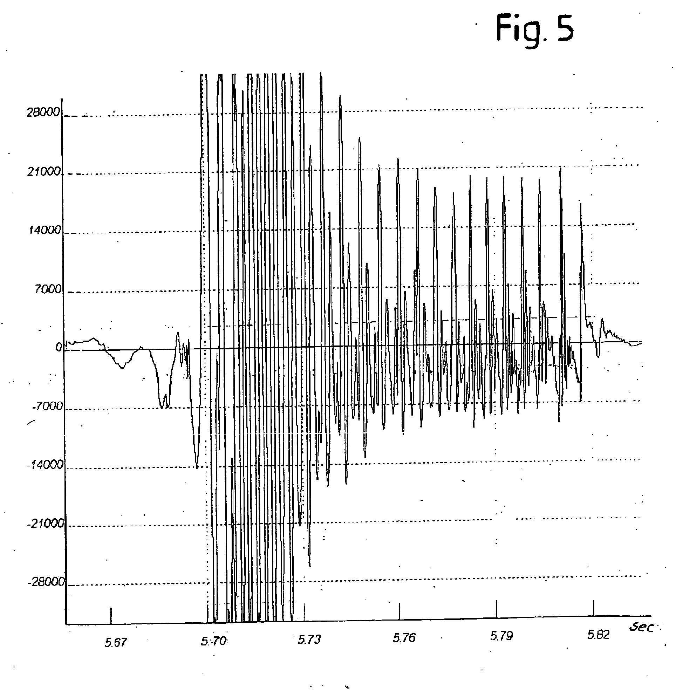

On the other hand, Fig.5 shows a recording in which, during the swallowing of a liquid bolus, a sound signal is generated that remains high for approximately 30 ms and is then gradually dampened for a total duration in the order of 100 ms, showing a slow and partial closing of the glottis corresponding to a pathological condition.

The computer is equipped with software suitable to highlight abnormalities with respect to normal readings.

It is understood that the drawing only shows an example given by way of a practical demonstration of the finding, as said finding can vary in forms and arrangements without however departing from the scope of the concept underlying the invention. Any reference numbers in the appended claims are provided to facilitate reading of the claims with reference to the description, and do not limit the scope of protection represented by the claims.

## Claims

1. An apparatus to detect dysphagia,
the apparatus comprising
- a collar (1) comprising a microphone (7) which, collar when worn correctly against the skin of a patient a holds sensitive part of the microphone (7) against the patient's glottis, the microphone (7) being connected to a recorder to record the sound produced by the passage of boluses transiting in the pharynx-esophagus-trachea;
- a computer provided with specific software suitable to analyze sound recordings corresponding to the sound recorded by said recorder to highlight deviations with respect to normal standard recordings, said collar (1) comprising a part (5) made of sound insulating material having the microphone (7) inserted therein,
wherein that said part (5) made of sound insulating material forms an extension projecting downward from an adjustable strap (3) of the collar (1).

2. Apparatus according to claim 1, **characterized in that** said extension has a symmetrial shape with respect to a vertical plane of symmetry of the patient, when said collar (1) is worn correctly.

3. Apparatus according to claims 1 or 2, **characterized in that** said collar (1) is composed of washable and sterilizable material.

4. Device according to claims 1 or 2, **characterized in that** said collar (1) is disposable.

## Patentansprüche

1. Vorrichtung zum Detektieren von Dysphagie, wobei die Vorrichtung umfasst
- einen Kragen (1), der ein Mikrofon (7) umfasst, wobei der Kragen, wenn er korrekt auf der Haut eines Patienten getragen wird, den empfindlichen Teil des Mikrofons (7) gegen die Glottis des Patienten hält, wobei das Mikrofon (7) mit einem Rekorder zum Aufzeichnen des Geräusches, das durch die Passage von Boli, die in Rachen-Speiseröhre-Luftröhre hindurch treten, erzeugt wird, verbunden ist;
- einen Computer, der mit spezifischer Software versehen ist, die zum Analysieren von Geräuschaufzeichnungen geeignet ist, die dem Geräusch entsprechen, das durch den Rekorder aufgezeichnet wurde, um Abweichungen hinsichtlich normaler Standardaufzeichnungen hervorzuheben,
wobei der Kragen (1) einen Teil (5) umfasst, der aus schalldämmendem Material hergestellt ist, und das Mikrofon (7) darin eingeführt ist,
wobei der Teil (5), der aus schalldämmendem Material hergestellt ist, eine Erstreckung bildet, die von einem einstellbaren Band (3) des Kragens (1) nach unten übersteht.

2. Vorrichtung gemäß Anspruch 1, **dadurch gekennzeichnet, dass** die Erstreckung eine symmetrische Form hinsichtlich einer vertikalen Symmetrieebene des Patienten aufweist, wenn der Kragen (1) korrekt getragen wird.

3. Vorrichtung gemäß Anspruch 1 oder 2, **dadurch gekennzeichnet, dass** der Kragen (1) aus waschbarem und sterilisierbarem Material aufgebaut ist.

4. Vorrichtung gemäß Anspruch 1 oder 2, **dadurch gekennzeichnet, dass** der Kragen (1) ein Einwegartikel ist.

## Revendications

1. Appareil pour détecter une dysphagie, l'appareil comprenant :
un collier (1) comprenant un micro (7), lequel collier, lorsqu'il est porté correctement contre la peau d'un patient, maintient une partie sensible du micro (7) contre la glotte du patient, le micro (7) étant raccordé à un enregistreur pour enregistrer le son produit par le passage des bolus transitant dans le pharynx-oesophage-trachée ;
un ordinateur doté d'un logiciel spécifique approprié pour analyser les enregistrements sonores correspondant au son enregistré par ledit enregistreur pour mettre en relief les déviations par rapport aux enregistrements standards normaux,
ledit collier (1) comprenant une partie (5) réalisée avec un matériau isolant acoustique ayant le micro (7) inséré à l'intérieur de ce dernier,
dans lequel ladite partie (5) réalisée avec le matériau isolant acoustique forme une extension en saillie vers le bas à partir d'une attache ajustable (3) du collier (1).

2. Appareil selon la revendication 1, **caractérisé en ce que** ladite extension a une forme symétrique par rapport à un plan vertical de symétrie du patient, lorsque ledit collier (1) est correctement porté.

3. Appareil selon les revendications 1 ou 2, **caractérisé en ce que** ledit collier (1) est composé d'un matériau lavable et stérilisable.

4. Dispositif selon les revendications 1 ou 2, **caractérisé en ce que** ledit collier (1) est jetable.
